# EUROPEAN PATENT SPECIFICATION

(11) **EP 0 755 382 B1**
(45) Date of publication and mention of the grant of the patent: **13.06.2001**
(21) Application number: 95915256.2
(22) Date of filing: 13.04.1995
(51) Int. Cl.: C07D 215/54, A61K 31/47

(54) **QUINOLONE DERIVATIVE FOR TREATMENT OF URINARY INCONTINENCE**
CHINOLONDERIVAT ZUR BEHANDLUNG DER HARNINCONTINENZ
DERIVE DE QUINOLONE UTILISE DANS LE TRAITEMENT DE L'INCONTINENCE URINAIRE

(30) Priority: 14.04.1994 GB 9407432
(43) Date of publication of application: 29.01.1997
(73) Proprietor: AstraZeneca AB, 151 85 Södertälje (SE)
(72) Inventor: WARAWA, John, Edward, Wilmington, DE 19808 (US); OHNMACHT, Cyrus, John, Jr., Wilmington, DE 19810 (US); TRAINOR, Diane, Amy, Wilmington, DE 19803 (US); HULSIZER, James, Michael, Wilmington, DE 19810 (US)
(74) Representative: Bill, Kevin
(86) International application number: GB9500845
(87) International publication number: WO9528388

(56) References cited:
- EP-A- 0 539 153
- WO-A-94/08966
- DE-A- 2 003 148

## Description

This invention relates to a novel compound which is useful in the treatment of bladder instability in mammals such as man. More specifically, this invention relates to (S)-(-)-4-(3-cyanophenyl)-2-trifluoromethyl-4,6,7,8-tetrahydro-5(1H)quinolone (the compound), the use of the compound in the treatment of urinary incontinence in mammals (including man), processes for preparing the compound and pharmaceutical compositions containing the compound.

The existing treatments for urinary incontinence are generally poor, relying on drugs that had originally been developed for other indications. One group of such drugs comprises the calcium channel blockers, such as nifedipine, which were originally developed and are primarily used as cardiovascular agents.

Nifedipine belongs to a structural class of compounds known as the dihydropyridines. This structural class has been extensively investigated, and the structural requirements for calcium blocking activity are now quite well established. Thus, as described on Chapter 14.1 of the medicinal chemistry text book, Comprehensive Medicinal Chemistry, Volume 3, Edited by John C. Emmett and published by Pergamon Press in 1990, the compounds possess a 1,4-dihydropyridine ring having, optimally, an aryl group at the 4-position and ester groups at the 3- and 5-positions. Removing the ester groups or replacing them with acetyl or cyano groups is associated with a reduction in activity. Generally, the compounds have methyl groups at the 2- and 6-positions.

Grinshteins et al, Khim. Geterotsikl. Soedin. (6), 1118-20, 1967 disclose the compounds 3-cyano-4-phenyl-2,7,7-trimethyl-4,6,7,8-tetrahydro-5(1H)-quinolone and 3-ethanoyl-4-phenyl-2,7,7-trimethy-l-4,6,7,8-tetrahydro-5(1H)-quinolone. Vitolinya et al, Khim.-Farm. Zh., 15(1), 39-42, 1981 discloses an investigation of the effect of several 4,6,7,8-tetrahydro-5(1H)-quinolones having an ester or cyano group at the 3-position on the cardiovascular system and on intestinal smooth muscle. 3-Cyano-4-phenyl-2,7,7-trimethyl-4,6,7,8-tetrahydro-5(1H)-quinolone is reported to possess hypotensive properties and to be capable of blocking the spasmogenic effect of both acetylcholine and barium chloride on intestinal smooth muscle.

DE 2003148 discloses a group of 1,4-dihydropyridine derivatives, including certain 4,6,7,8-tetrahydro-5(1H)-quinolones, which possess an ester or keto group at the 3-position and which are said to display a wide and multi-faceted pharmacological spectrum of action. The main effects said to be displayed by the compounds include strong muscular spasmoytic effects which become evident in the smooth musculature of the gastrointestinal tract, of the urogenital tract and of the respiratory system. Other main effects are stated to be on the heart (a "heart-relieving" effect) and in reducing the blood pressure of normotonic and hypertonic animals, so that they can be used as antihypertensive agents.

It is known that bladder tissue is excitable and that urinary incontinence can be caused by uncontrolled or unstable bladder contractions. A compound has been found that is unexpectedly capable of relaxing bladder smooth muscle, thus preventing or ameliorating uncontrolled or unstable bladder contractions. Hence, the compound may be useful for the treatment of urge incontinence, which includes for example detrusor instability, which may result from cystitis, urethritis, tumors, stones, diverticuli or outflow obstruction; and detrusor hyperreflexia, which may result from stroke, dementia, parkinsons, suprasacral spinalcord injury or suprasacral spinalcord disease.

It has also unexpectedly been found that the compound is a potassium channel opener. It is known that by functioning to open potassium channels, potassium channel opening compounds can thereby function to relax smooth muscle. Virile not wishing to be bound by theory, it is accordingly believed that the compound functions by opening potassium channels in bladder cells and thereby relax bladder smooth muscle tissue, thus preventing or ameliorating uncontrolled bladder contractions which can cause urinary incontinence. Nurse D. A., Restorick J.H., and Mundy A.R., British Journal of Urology, (1991), 68, 27-31 discloses that cromakalim, which is well known as a potassium channel opener, has been found to be effective in a preliminary clinical trial for the treatment of urinary incontinence.

Accordingly, the invention provides the compound (S)-(-)4-(3-cyanophenyl)-2-trifluoromethyl-4,6,7,8-tetrahydro-5(1H)-quinolone and pharmaceutically acceptable salts thereof.

It will be appreciated that the compound may exhibit polymorphism and may form solvates. It is to be understood that the present invention encompasses any polymorphic form, or solvate, or mixtures thereof, which is useful for relaxing bladder smooth muscle, it being well known in the art how to determine whether a compound is capable of relaxing bladder smooth muscle, by the standard tests described hereinafter. The stereochemistry of the compound was firstly noted as (-), since then the absolute stereochemistry has been found to be (S). Thus, it may be preferred to use the compound in a form which is characterized as containing, for example, at least 95%, 98% or 99% enantiomeric excess (ee) of the (S)-(-)-form.

The compound can be made by processes which include processes known in the chemical arts for the production of structurally analogous compounds. Such processes for the manufacture of (S)-(-)-4-(3-cyanophenyl)-2-trifluoromethyl-4,6,7,8-tetrahydro-5(1H)quinolone are provided as further features of the invention and are illustrated by the following procedures. Such a process can be effected, generally, by decarboxylation of a corresponding (S)-(-)-form carboxylic acid of formula II.

The decarboxylation reaction can conveniently be carried out at an elevated temperature for example in the range of from 50°C to 250°C, preferably, in the range of from 190°C to 220°C without an acid catalyst, especially 130°C to 185°C (for example, using a heating mantle) and preferably, in the range of from 90°C to 120°C with an acid catalyst. The non-acid catalyzed decarboxylation reaction can be carried out as a neat melt, or in an inert solvent of appropriate boiling point such as diphenyl ether or N-methyl-pyrrolidin-2-one. A preferred solvent is N-methylpyrrolidin-2-one. Suitable solvents for the acid catalyzed decarboxylation include alcohols, for example, methanol or ethanol; dimethylsulfoxide; aromatic hydrocarbons such as toluene; ethers, such as for example 1,2-dimethoxyethane or diglyme; and N-methylpyrrolidin-2-one. Concentrated sulphuric acid, phosphoric acid, hydrochloric acid, hyrdrobromic acid, hydriodic acid, strong organic acids such as trifluororacetic acid, organic sulphonphonic acid such as methanesulphonic acid or p-toluenesulphonic acid may conveniently be used as an acid catalyst. The reaction can be conveniently carried out neat at a temperature at or above the melting point of the material.

The (S)-(-)-form carboxylic acid of formula II is conveniently prepared by resolution of the corresponding racemic carboxylic acid of formula II. A convenient method of resolving the compound of formula II is to form a resolved salt of the acid, for example, using S-(-)-α-methylbenzylamine. The salt is retrieved and then acidified to release the resolved carboxylic acid of formula II. The recrystallisations are preferably carried out at a temperature of 75°C or lower and preferably without agitation.

Alternately, crude racemic compound of formula I may be resolved using a chiral column to give the compound of formula I.

If not commercially available, the materials for the necessary processes, such as those described following, may be made by procedures which are selected from standard organic chemical techniques, techniques which are analogous to the synthesis of known, structurally similar compounds, or techniques which are analogous to the above described procedure or the procedures described in the examples.

An intermediate of formula II can be prepared as shown in Scheme I, by reacting an acetoacetic ester of formula III or hemiacetals thereof, in which ORa is an alcohol residue, which forms an ester that can be cleaved under mild basic conditions, such as for example a 2-cyanoethoxy ester, with 3-cyanobenzaldehyde and 1,3-cyclohexanedione to give an alcohol of formula IVa. Dehydration of the alcohol yields an ester of formula IV, which can be saponified to give an acid of formula II.

An intermediate of formula II can alternatively be prepared as shown in Scheme II, by reacting an acetoacetic ester of formula III or hemicacetals thereof in which 0Ra is an alcohol residue, which forms an ester that can easily be cleaved by acid, such as for example an isobornyl ester, with 3-cyanobenzaldehyde and 1,3-cyclohexanedione to give an alcohol of formula IVa.

Acid catalyzed dehydration and hydrolysis of a compound of formula IVa, for example under conditions similar to those described the Examples, yields a compound of formula II. The acid catalyst for preparation of the compound of formula IV from IVa may be any of the acid catalysts hereinbefore mentioned and is preferably p-toluenesulphonic acid. The acid catalyst for preparation of the compound of formula II from IVa is preferably p-toluenesulphonic acid. The reaction is preferably performed in presence of a inert solvent such as toluene or acetic acid. In order to prevent decarboxylation, the solvent is advantageously anhydrous, for example, glacial acetic acid.

To further minimise decarboxylation, the temperature of the reaction should be maintained at a maximum of 104°C, preferably in the range from 100°C to 104°C, especially 102°C to 104°C.

The isobornyl ester can be prepared by ester interchange of isobornyl and ethyl 4,4,4-trifluoroacetoacetate and 2-cyanoethyl hemiacetal of 2-cyanoethyl 4,4,4-trifluoroacetoacetate by ester interchange of 3-hydroxypropionitrile and ethyl 4,4,4-trifluoroacetoacetate.

The processes for preparation of the compound of formula I and the intermediates therefor comprise further aspects provided by the present invention.

Pharmaceutically acceptable salts may be obtained using standard procedures well known in the art, for example by reacting the compound with a suitable acid or base affording a physiologically acceptable counterion.

When used to treat urinary incontinence, the compound is generally administered as an appropriate pharmaceutical composition which comprises the compound together with a pharmaceutically acceptable diluent or carrier, the composition being adapted for the particular route of administration chosen. Such compositions are provided as a feature of the invention.

According to another aspect, therefore, the invention provides a pharmaceutical composition, which comprises the compound or a pharmaceutically acceptable salt thereof, as defined hereinabove, and a pharmaceutically acceptable diluent or carrier.

The compositions may be obtained employing conventional procedures and excipients and binders and may be in a variety of dosage forms. For example, they may be in the form of tablets, capsules, solutions or suspensions for oral administration; in the form of suppositories for rectal administration; in the form of sterile solutions or suspensions for administration by intravenous, intravesicular, subcutaneous or intramuscular injection or infusion; or in the form of a patch for transdermal administration.

The invention further provides a method for the treatment of urinary incontinence, comprising administering to a mammal in need of such treatment an effective amount of the compound or a pharmaceutically acceptable salt thereof.

Treatment using the compound can be remedial or therapeutic as by administering a compound following the onset or development of urinary incontinence in a patient. Treatment can also be prophylactic or prospective by administering the compound in anticipation that urinary incontinence may develop, for example in a patient who has suffered from incontinence in the past.

According to a further aspect, the invention provides the use of the compound in the manufacture of a medicament for the treatment of urinary incontinence.

Because the compound functions to open cell potassium channels, it may also be useful as a therapeutic agent in the treatment of other conditions or diseases in which the action of a therapeutic agent which opens potassium channels is desired or is known to provide amelioration. Such conditions or diseases include hypertension, asthma, peripheral vascular disease, right heart failure, congestive heart failure, angina, ischemic heart disease, cerebrovascular disease, glaucoma, renal cholic, disorders associated with kidney stones, irritable bowel syndrome, male pattern baldness, premature labor, and peptic ulcers.

The dose of the compound which is administered will necessarily be varied according to principles well known in the art taking account of the route of administration, the severity of the incontinence condition, and the size and age of the patient. In general the compound will be administered to a warm blooded animal (such as man) so that an effective dose is received, generally a daily dose of above 0.005, for example in the range of about 0.01 to about 10 mg/kg body weight. Preferably the compound is administered orally in this dose range.

It will be apparent to those skilled in the art that the compound can be co-administered with other therapeutic or prophylactic agents and/or medicaments that are not medically incompatible therewith.

The actions of the compound as a smooth muscle relaxant useful as a therapeutic agent for the treatment of urinary incontinence through its action to open potassium channels and hyperpolarize the membrane potential in the bladder detrusor smooth muscle can be shown using suitably designed in vitro tests, such as the one described following. The compound exhibits an IC₅₀ of 4.2 micromolar in the test. "IC₅₀" is a well understood term and means the concentration of test compound which causes a 50% decrease in the in vitro contraction of the bladder tissue described in the following test.

Hale albino Hartley guinea pigs (450-500g) are sacrificed by carbon dioxide induced asphyxiation and quickly exsanguinated. The lower abdominal cavity is opened and the urinary bladder isolated.
The bladder is cleaned of surrounding connective and adipose tissue, and the portion above the ureteral orifices is removed and washed in Krebs-Henseleit buffer solution of the following composition (in mH): NaCl 118.0, KCl 4.7, HgSO₄ 1.2, KH₂PO₄ 1.2, CaCl₂ 2.5, NaHCO₃ 25.0 and d-glucose 11.1. The solution is warmed to 37°C and gassed with 95% O₂ and 5% CO₂. With vigorous bubbling, the solution should have a pH value close to 7.4.

The dome of the washed bladder is cut off and discarded; the remaining bladder is placed on a gauze in a Petri dish containing the buffer solution. A mid-ventral longitudinal cut is made with scissors to open the bladder. The strips cut from the dome and the base edge are discarded. The remaining detrusor mid-section is cut into two horizontal strips with an approximate width of 2.0 mm. These two strips are further bisected at the mid-dorsal section, creating four strip of similar dimensions. Each strip thus contains both dorsal and ventral portions of the bladder.

The two ends of each individual strip are tied to a glass support rod and a force-displacement transducer (Grass model FR03), respectively, with 4-0 black braided silk suture.

The transducers are connected to a polygraph (Grass model 7E), which is calibrated at 5 mV/cm and the calibration checked for linearity with weights of 5 and 0.5 grams. The analog electrical output signals from the polygraph are digitized by a Modular Instrument Micro 5000 signal processing system using Biowindow Data Acquisition Software, which is run under the Microsoft OS/2 operating system with an IBM-compatible PC.

The detrusor strips on the glass rod are secured in 20 ml tissue baths and allowed to equilibrate under a preload tension of 2 grams. During the following 45 to 60 min equilibration period, the tissue is washed with fresh buffer solution at 15 min interval, with the tension adjusted, if necessary, to 2 grams prior to washing.
After the equilibration period, a priming dose of 15mM KCl (total concentration in the bath) is applied. The tissue is washed after 10 min and washed twice more at 15 min intervals with tension adjusted to 2 grams before each washing.

When the tissue relaxes to a steady state after the final washing, 15 mM KCl is again applied. Once the myogenic activity of the tissue reaches a steady state, the baseline data are acquired through the Biowindows Data Acquisition System by averaging 5 min of the myogenic data sampled at 32 Hz. Once the baseline is acquired, the experimental compound is dosed in a cumulative manner in half log unit increments. The contact time for each dose is 10 min with the final 5 min being the period of time that the dose reponse data are required. If 30 µM of the test compound does not abolished the detrusor mechanical activity, then 30 µM cromakalim, a putative potassium channel opener, is dosed to establish a maximum response. The effect of the compound at each dose is expressed as % of the maximum inhibitory response, which is further normalized with respect to the corresponding effect of the compound vehicle control. The normalized response is then used to derive the IC₅₀ of the relaxant activity of the compound through the application of Marquardt's nonlinear iterative curve fitting technique to a standard dose-response function.

The ability of the compound to open potassium channels in detrusor smooth muscle can be further demonstrated by a second in vitro test. This second in vitro test is similar to the one described above with regard to tissue preparation and data acquisition.
However, the following exceptions are noted. In this second test, the contraction of the detrusor strips during priming and after the equilibration period is achieved with 80 mM instead of 15 mM KCl (total concentration in the bath). A sustained tension in the tissue is evident after this high KCl stimulation, because voltage-sensitive calcium channels have been rendered open to permit an influx of calcium into the cells and the development of tonic tension. This tension is totally abolished with 300 µM of papaverine, which is thereby used to establish the maximum response in this test.

Typical calcium channel blockers like nifedipine, nimodipine, isradipine, and verapamil are able to relax and reduce the myogenic activity of guinea pig detrusor strips in both tests by virtue of their blocking action on calcium channels. However, all of the aforementioned calcium channel blockers are more potent in the second test when 80 mM KCl is used, than in the first test where 15 mM KCl is used. In contrast, while the putative potassium channel opener cromakalim has a potent relaxant activity in the first test with an IC₅₀ in the range of 0.6 to 0.9 µM, it demonstrates insignificant relaxant activity in the second test at concentrations as high as 30 µM. Thus, the profile of a higher relaxant activity in the first test than in the second of compounds according to the invention indicates that the compounds are functioning as potassium channel openers. The compound exhibits an IC₅₀ of 41.1 uM in the second test.

The ability of the compound to act as a potassium channel opener on bladder tissue may be further demonstrated by a standard test which measures the effect of test compounds on the rate of efflux of rubidium (⁸⁶Rb) or potassium (⁴²K) from the tissue.

It will be further appreciated by those skilled in the art that the efficacy of the compound can be demonstrated by standard assays in vivo. The following is a description of such a standard test, which can be used to ascertain if a test compound is active and, additionally, if a test compound exhibits selectivity for the bladder without significant cardiovascular effects when dosed orally.

Hale Wistar rats (400 - 500 g) are anesthetized with 50 mg/kg Nembutal, i.p. For each rat, the abdominal region and the front and back of the neck are shaved and povidone-iodine is applied to the skin. For carotid catheterization, the left carotid artery is exposed via a small ventral cervical incision. The exposed area is flushed with a 2% lidocaine HCl solution to relax the vessel. The catheter, filled with 0.9% saline, is introduced approximately 2.4 cm into the artery so that its tip resided in the aortic arch. The distal end of the catheter is exteriorized at the nape of the neck, filled with heparin (1000 units/ml) and heat sealed. For bladder catheterization, the bladder is exposed through a midline abdominal incision. A trocar is passed through the abdominal muscle about 1 cm from the upper end of the incision and then tunneled subcutaneously to emerge through the skin at the back of the neck. A saline-filled catheter is passed through the trocar. A small opening in the bladder dome is created with an Accu-Temp cautery. The catheter is placed into the bladder and secured with a 4-0 silk ligature. The catheter is flushed with saline and patency is noted. The external end of the catheter is heat-sealed to prevent urine leakage. The abdominal muscles and the skin are sutured. Both catheters are threaded through a stainless steel anchor button (Instech), which is then sutured to the subcutaneous muscle at the point of exteriorization. The skin is sutured closed over the button. The animals are allowed to recover from anesthesia.

24 - 48 hours after surgery, each rat is placed in a metabolism cage and connected via the anchor button to an Instech spring tether and swivel system to protect the catheters from damage and to allow the animal free movement in the cage. The carotid catheter is connected to a Gould P23XL pressure transducer for blood pressure measurement. The bladder catheter is connected to a pump for saline infusion and to a pressure transducer by means of PE50 tubing and a 4-way stopcock. A toploading balance with a collection cup is placed under the cage for urine output measurement.

The rats are weighed, orally sham-dosed (dosing needle introduced, but no fluid expelled), and transvesical saline infusion (.18 ml/min) is begun and continued throughout the experiment. Variations in blood pressure, heart rate, intravesical pressure and urine output are recorded on either a Grass Polygraph or a Gould TA4000 recording system. The animals are allowed to equilibrate until the micturition pattern becomes consistent (approx. 45 - 90 min.). At this point, a basal level of each experimental parameter is recorded and the rats are administered by oral gavage the appropriate dose of compound (in a 75% PEG 400 - saline vehicle) in concentrations such that the volume is 1 ml/kg body weight. The effects of the compounds on experimental parameters are followed for five hours after administration.

Experimental results for both the interval between contractions and also heart rates are expressed as the mean ± S.E.M. (Standard Error of Measures) % change from basal level, with each animal serving as its own control. MAP is expressed as mean ± S.E.M mm Hg change from basal level.

The compound according to the invention is active in the above described tests being active and selective for the bladder without significant cardiovascular effects, when dosed orally, for example, at 3 mg/kg in the above in vivo screen.

The invention will now be illustrated by the following non-limiting examples in which, unless stated otherwise:
(i) temperatures are given in degrees Celsius (°C); operations were carried out at room or ambient temperature, that is, at a temperature in the range of 18-25 °C;
(ii) organic solutions were dried over anhydrous magnesium sulfate; evaporation of solvent was carried out using a rotary evaporator under reduced pressure (600-4000 pascals; 4.5-30 mm Hg) with a bath temperature of up to 60 °C;
(iii) chromatography means 'flash chromatography; reversed phase chromatography means flash chromatography over octadecylsilane (ODS) coated support having a particle diameter of 32-74 µ, known as "PREP-40-ODS" (Art 731740-100 from Bodman Chemicals, Aston, PA, USA); Thin layer chromatography (TLC) was carried out on silica gel plates;
(iv) in general, the course of reactions was followed by TLC and reaction times are given for illustration only;
(v) melting points are uncorrected and (dec) indicates decomposition; the melting points given are those obtained for the materials prepared as described; polymorphism may result in isolation of materials with different melting points in some preparations;
(vi) final products had satisfactory proton nuclear magnetic resonance (NMR) spectra;
(vii) yields are given for illustration only and are not necessarily those which may be obtained by diligent process development; preparations were repeated if more material was required;
(viii) when given, NMR data is in the form of delta values for major diagnostic protons, given in parts per million (ppm) relative to tetramethylsilane (TMS) as an internal standard, determined at 300 MHz using perdeuterio dimethyl sulfoxide (DHSO-d₆) as solvent; conventional abbreviations for signal shape are used; coupling constants (J) are given in Hz; Ar designates an aromatic proton when such an assignment is made;
(ix) chemical symbols have their usual meanings; SI units and symbols are used;
(x) reduced pressures are given as absolute pressures in pascals (Pa); elevated pressures are given as gauge pressures in bars;
(xi) solvent ratios are given in volume:volume (v/v) terms; and
(xii) mass spectra (MS) were run with an electron energy of 70 electron volts in the chemical ionization (CI) mode using a direct exposure probe; where indicated ionization was effected by electron impact (EI) or fast atom bombardment (FAB); values for m/z are given; generally, only ions which indicate the parent mass are reported.

### Example 1. (S)-(-)-4-(3-Cyanophenyl)-2-trifluoromethyl-4,6,7,8-tetrahydro-5(1H)-quinolone.

A stirred solution of (S)-(-)-4-(3-cyanophenyl)-2-trifluoromethyl-5-oxo-1,4,5,6,7,8-hexahydroquinoline-3-carboxylic acid (3.93g, 10.85 mmol) in N-methylpyrrolidin-2-one (24mL) was placed in a preheated oil bath at 210°C for 20 minutes. The cooled reaction mixture was then poured into water (200mL) and extracted twice with ethyl ether. The combined ether extracts were washed twice with water, dried (MgSO₄), filtered and the solvent removed to yield an off-white solid. Chromatography (eluent: methylene chloride/ether 9:1) and trituration with ethyl ether/hexane provided the title compound (3.42g, 79%) as a white solid mp 187-189°C. NMR: 1.88-1.91 (m, 2H, CH₂), 2.21-2.25 (m, 2H, CH₂), 2.53-2.64 (m, 2H, CH₂), 4.68(d, 1H, J=5.3 Hz, CH), 5.61 (d, 1H, J=5.3Hz, CH), 7.50-7.54 (m, 2H, Ar), 7.61-7.66 (m, 2H, Ar), 9.42 (s, 1H, NH); MS: m/z=319(M+1); [α]_{D}²³=-606.8° (c = 0.665, methanol). Analysis for C₁₇H₁₃F₃N₂O: Calculated: C, 64.14; H, 4.12; N, 8.80; Found: C, 64.07; H, 4.24; N, 8.78.

¹⁹F-NMR analysis of this material in the presence of the chiral shift reagent (R)-(-)-1-(9-anthryl)-2,2,2-trifluoroethanol-d₁₁ (CDCl₃ at -30°C) showed the (S)-(-)-enantiomer to be present in approximately 99% ee.

The intermediate (S)-(-)-4-(3-cyanophenyl)-2-trifluoromethyl-5-oxo-1,4,5,6,7,8-hexahydroquinoline-3-carboxylic acid was prepared as follows.

### a. (±)-Isobornyl 4,4,4-trifluoroacetoacetate.

A stirred mixture of ethyl 4,4,4-trifluoroacetoacetate (160.28g 870 mmol) and (±)-isoborneol (86.81g 563 mmol) was stirred at 130°C (bath temperature) for 18 hours under a 4 inch Vigreaux column which allowed ethanol to distill off. The bath temperature was then increased to 150°C to distill the residual ethanol; a total of 29 mL (88% of theory) was collected. The remaining mixture was then fractionated at reduced pressure to yield (±)-isobornyl 4,4,4-trifluoroacetoacetate as a colorless oil (124.6g 76%); bp 84-92°/0.4 torr; NMR (CDCl₃): 0.82-1.17 (m, 11H, CH₃, CH₂) 1.55-1.84 (m, 5H, CH₂, CH) 3.72 (s, diketo form CH₂) 4.74-4.81 (m, 1H, -OCH) 5.59 (s, enol form CH) 11.97 (s, enol form OH).

### b. (±)-Isobornyl 4-(3-cyanophenyl)-2-trifluoromethyl-2-hydroxy-5-oxo-1,2,3,4,5,6,7,8-octahydroquinoline-3-carboxylate.

A stirred mixture of (±)-isobornyl 4,4,4-trifluoroacetoacetate (82.6g 282.8 mmol), 1,3-cyclohexanedione (31.7g 282.8 mmol), 3-cyanobenzaldehyde (37.1 g 282.8 mmol), and ammonium acetate (54.4g 706.3 mmol) in ethanol (2070 mL) was refluxed for 4 hours. After removal of precipitated 9-(3-cyanophenyl)-3,4,6, 7,9,10-hexahydro-1,8-(2H,5H)-acridinedione by filtration, the filtrate was concentrated in vacuo. Chromatography (eluent: ethyl acetate/hexane 7:3) provided (±)-isobornyl 4-(3-cyanophenyl)-2-trifluoromethyl-2-hydroxy-5-oxo-1,2,3,4,5,6,7,8-octahydroquinoline-3-carboxylate 92g (63%) as a white solid; mp 209-213°C dec; NMR: 0.63-0.73 (m, 9H, CH₃), 0.86-1.64 (m, 7H, CH₂, CH), 1.84-1.88 (m, 2H, CH₂), 2.03-2.07 (m, 2H, CH₂), 2.34-2.63 (m, 2H, CH₂) 2.64-2.72 (m, 1H, CH), 3.92-3.96 (d, 1H, CH), 4.10-4.16 (m, 1H, CH), 7.22-7.25 (d, 1H, OH), 7.39-7.45 (m, 2H, Ar), 7.49 (s, 1H, Ar), 7.57-7.61 (m, 1H, Ar), 8.11-8.13 (d, 1H, Ar); MS: m/z=517(H+1). Analysis for C₂₈H₃₁F₃N₂O₄: Calculated: C; 65.10; H; 6.05; N; 5.42; Found: C; 64.93; H; 6.05; N; 5.22.

### c. 4-(3-Cyanophenyl)-2-trifluoromethyl-5-oxo-1,4,5,6,7,8-hexahydroquinoline-3-carboxylic acid.

A stirred mixture of (±)-isobornyl 4-(3-cyanophenyl) -2-trifluoromethyl-2-hydroxy-5-oxo-1,2,3,4,5,6,7,8-octahydroquinoline -3-carboxylate (8.5g 16.5 mmol), p-toluenesulfonic acid (1.05 g 5.5 mmol) and toluene (170 mL) was refluxed for 3 hours. The reaction mixture consisted of an insoluble gum which contained the desired acid and toluene solution. To prevent decarboxylation, only enough heat was applied to the reaction mixture to obtain a mild reflux. After removal of solvent, the residue was partitioned between ethyl acetate and water. The ethyl acetate was washed with water, separated and extracted twice with saturated aqueous sodium bicarbonate. The stirred combined sodium bicarbonate extracts were cooled in an ice bath and concentrated hydrochloric acid was added dropwise until the solution was strongly acidic. The mixture was extracted with ether and the ether layer was dried, filtered, and concentrated in vacuo to give a yellow oily gum. Trituration with methylene chloride/hexane yielded the carboxylic acid (1.8g, 31%) as an off-white solid. The material was identical by NMR and tlc (silica gel - 10% methanol in chloroform containing a few drops of acetic acid) to the material described and characterized in sub-part i. of Example 1.

The ethyl acetate layer was dried, and evaporated, to obtain impure (±)-isobornyl 4-(3-cyanophenyl)-2-trifluoromethyl-5-oxo-1,4,5,6,7,8-hexahydroquinoline-3-carboxylate, which was identical to a previously prepared and characterized sample; white solid; mp 179-180°C; NMR: 0.61-0.72(m, 9H, CH₃), 1.00-2.6 (m, 13H, CH₂, CH), 4.45-4.57 (m, 1H, CH), 4.88-4.92 (d, 1H, CH), 7.46-7.55 (m, 3H, Ar), 7.65-7.69 (m, 1H, Ar), 9.70-9.71 (d, 1H, NH). HS(CI, CH₄): 517 (M+1). Analysis for C₂₈H₂₉F₃N₂O₄: Calculated: C; 67.46; H; 5.86; N; 5.62; Found: C; 67.29; H; 5.99; N; 5.71.

The recovered (±)-isobornyl 4-(3-cyanophenyl)-2-trifluoromethyl-5-oxo-1,4,5,6,7,8-hexahydroquinoline-3-carboxylate was subjected to conditions similar to those described in Example 1.c. to yield an additional 1.1g of 4-(3-cyanophenyl)-2-trifluoromethyl-5-oxo-1,4,5,6,7,8-hexahydroquinoline-3-carboxylic acid, for a total yield of 2.9g (49%).

### d. S-(-)-α-Methylbenzylamine (S)-(-)-4-(3-cyanophenyl)-2-trifluoromethyl-5-oxo-1,4,5,6,7,8-hexahydroquinoline-3-carboxylic acid salt.

To a stirred solution of racemic 2-trifluoromethyl-4-(3-cyanophenyl)- 5-oxo-1,4,5,6,7,8-hexahydroquinoline-3-carboxylic acid (28.2g, 77.84 mmol) in n-butanol (211 mL) was added toluene (1000 mL) followed by a solution of S-(-)-α-methylbenzylamine (9.4g, 77.84 mmol) in toluene (198 mL). After standing at ambient temperature overnight, the resulting precipitate was filtered, and washed with toluene and ethyl ether to yield 32 g of a white salt. Five recrystallizations from toluene/n-butanol (3:1), heating the mixture until the salt dissolves, provided the salt as a white solid (7.77g); mp softens and turns glasslike at 112-115°C, liquid melt 148-150°C; NMR: 1.36-1.38 (d, 3H, CH₃), 1.75-1.88 (m, 2H, CH₂), 2.19-2.23 (m, 2H, CH₂), 2.49-2.61 (m, 2H, CH₂), 3.35-3.42 (m, exchangeables, water) 4.24-4.26 (q, 1H, J=6.8Hz, CH), 4.91 (s, 1H, CH), 7.16-7.60 (m, 9H, Ar); MS: m/z=363(M+1); [α]_{D}=-180.5° (c=1.075, methanol, 23°C); 99%ee by ¹⁹F-NMR in CDCl₃. Analysis for C₂₆H₂₄F₃N₃O₃·1.0 C₄H₉OH·0.5 H₂O: Calculated: C, 63.59; H, 6.23; N, 7.42; Found: C, 63.66; H, 6.15; N, 7.06.

Further experimentation demonstrated that the maximum temperature during recrystallization should be 75°C to prevent decarboxylation and that agitation should not be applied during recrystallisation otherwise enantiomeric enhancement is poor.

### e. (S)-(-)-4-(3-Cyanophenyl)-2-trifluoromethyl-5-oxo- 1,4,5,6,7,8-hexahydroquinoline-3-carboxylic acid.

To a cooled (ice bath) stirred slurry of S-(-)-a-methylbenzylamine (S)-(-)-4-(3-cyanophenyl)-2- trifluoromethyl-5-oxo-1,4,5,6,7,8-hexahydroquinoline-3-carboxylic acid salt (7.5g, 15.5 mmol) in water (150 mL) was added concentrated hydrochloric acid dropwise until the mixture was strongly acidic. The mixture was extracted twice with ethyl ether, and the combined ether layer was dried, filtered and the solvent removed to yield a yellow foam. Trituration with dichloromethane returned 4.9g (88%) of the carboxylic acid as a pale yellow solid; mp 206-208°C; NMR: 1.74-1.95 (m, 2H, CH₂), 2.18-2.32 (m, 2H, CH₂), 2.54-2.73 (m, 2H, CH₂), 4.92 (s, 1H, CH), 7.47-7.52 (m, 3H, Ar), 7.64-7.68 (m, 1H, Ar), 9.60 (s, 1H, NH), 13.07(s, 1H, C00H); MS: m/z=363(H+1).

The intermediate 4-(3-cyanophenyl)-2-trifluoromethyl-5- oxo-1,4,5,6,7,8-hexahydroquinoline-3-carboxylic acid described at Example 1.c. can alternatively be prepared as described in Example 6.

### f. 2-Cyanoethyl hemiacetal of 2-cyanoethyl 4,4,4-trifluoroacetoacetate.

A mixture of ethyl 4,4,4-trifluoroacetoacetate (30 mL 205 mmol) and 3-hydroxypropionitrile (7.11g 100 mmol) was stirred at 150°C (bath temperature) for 18 hours under a 4 inch Vigreaux column which allowed ethanol to distill off. The remaining mixture was then fractionated at atmospheric pressure. The fraction distilling at 222-228 °C (11.71g) was determined to contain approximately 50 mole% of the 2-cyanoethyl hemiacetal of 2-cyanoethyl 4,4,4-trifluoroacetoacetate by NMR and mass spectral analysis; NMR (CDCl₃): 2.43-2.65 (m, 2H) 2.76-2.99 (m, 4H) 3.78-3.82 (m, 1H) 4.13-4.15 (m, 1H) 4.33-4.57 (m, 2H) 5.96 (s, 1H, OH).

### g. 2-Cyanoethyl 4-(3-cyanophenyl)-2-trifluoromethyl-2-hydroxy- 5-oxo-1,2,3,4,5,6,7,8-octahydroquinoline-3-carboxylate.

A stirred mixture of the material from Example 1.f. along with 3-cyanobenzaldehyde (7.34g 56 mmol), 1,3-cyclohexanedione (6.83g 56 mmol) and ammonium acetate (13.10g 170 mmol) in ethanol (400 mL) was stirred at reflux for 10 hours. The cooled solution was filtered to remove precipitated 9-(3-cyanophenyl)-3,4,6,7,9,10 -hexahydro-1,8-(2H,5H)-acridinedione. The filtrate was evaporated to dryness and the residue chromatographed (eluent methylene chloride, 1:1 ethyl acetate/methylene chloride and ethyl acetate) to yield 4.14 g of 2-cyanoethyl 4-(3-cyanophenyl)-2-trifluoromethyl-2-hydroxy-5-oxo- 1,2,3,4,5,6,7,8-octahydroquinoline-3-carboxylate; NMR: 1.86-1.90 (m, 2H, CH₂) 1.94-2.18 (m, 2H, CH₂) 2.31-2.38 (m, 1H, aliphatic) 2.50-2.72 (m, 3H, aliphatic) 2.82 (d, 1H, aliphatic, J=11.9) 3.35 (br.s, 1H, aliphatic) 3.94-4.02 (m, 2H, aliphatic) 7.37-7.59 (m, 5H, Ar, OH) 8.16 (s, 1H, NH); MS: m/z=434(M+1).

### h. 2-Cyanoethyl 4-(3-cyanophenyl)-2-trifluoromethyl-5-oxo- 1,4,5,6,7,8-hexahydroquinoline-3-carboxylate.

A mixture of 2-cyanoethyl 4-(3-cyanophenyl)-2--trifluoromethyl-2-hydroxy-5-oxo-1,2,3,4,5,6,7,8-octahydroquinoline-3 -carboxylate (4.14g 9.6 mmol), p-toluenesulfonic acid (0.61g 3.2 mmol) and toluene (100 mL) was stirred at reflux under a Dean-Stark apparatus for 2 hours. The reaction mixture, which consisted of dark oil and toluene phases, was cooled and poured on a 1.5 inch diameter chromatography column containing 90g of silica gel. The mixture was washed on the column with a little ethyl acetate. Elution with ethyl ether and trituration of the resulting solid with ethyl ether yielded the ester as a yellow-orange solid (2.84g , 72%); mp 148-151.5 °C; NMR: : 1.79-1.99 (m, 2H, CH₂) 2.19-2.33 (m, 2H, CH₂) 2.50-2.74 (m, 2H, CH₂) 2.85 (t, 2H, CH₂) 4.15-4.27 (m, 2H, CH₂) 4.94 (s, 1H, CH) 7.47-7.63 (m, 3H, Ar) 7.65-7.67 (m, 1H, Ar) 9.84 (s, 1H, NH); MS: m/z=416(M+1). Analysis for C₂₁H₁₆F₃N₃O₃: Calculated: C, 60.72; H, 3.88; N, 10.12; Found: C, 60.63; H, 3.80; N, 9.89.

### i. 4-(3-Cyanophenyl)-2-trifluoromethyl-5-oxo-1,4,5,6,7,8- hexahydroquinoline-3-carboxylic acid.

To a cooled (ice bath) stirred slurry of 2-cyanoethyl 2-trifluoromethyl-4-(3-cyanophenyl)-5-oxo-1,4,5,6,7,8- hexahydroquinoline-3-carboxylate (2.80g 6.74 mmol) in 1,2-dimethoxyethane (8.5 mL) was added dropwise over a 10 minute period a pre-cooled solution of sodium hydroxide (0.80g 20.0 mmol) in water (6.5 mL). A dark brown solution was obtained as starting ester dissolved. After stirring at room temperature for 2 hours the yellowish-brown solution was diluted with water (16 mL), returned to the ice bath and stirred as the mixture was treated with concentrated hydrochloric acid (2 mL). A brown oil precipitated which upon stirring solidified to a cream colored solid. The solid was filtered and washed with cold water. The material was dried at 50°/0.1 torr overnight to give the carboxylic acid (2.33g, 95%); mp 209-211°C dec. with gas evolution; NHR: 1.75-1.88 (m, 1H, CH₂) 1.89-1.95 (m, 1H, CH₂) 2.18-2.32 (m, 2H, CH₂) 2.54-2.73 (m, 2H, CH₂) 4.92 (s, 1H, CH) 7.47-7.54 (m, 3H, Ar) 7.65-7.67 (m, 1H, Ar) 9.62 (s, 1H, NH) 13.10 (s, 1H, CO₂H); MS: m/z=363(M+1). Analysis for C₁₈H₁₃F₃N₂O₃: Calculated: C, 59.67; H, 3.62; N, 7.73; Found: C, 59.53; H, 3.84; N, 7.69.

### Example 2. (S)-(-)-4-(3-Cyanophenyl)-2-trifluoromethyl-4,6,7,8- tetrahydro-5(1H)-quinolone.

The product of Example 1 was prepared on a larger scale as follows:
A stirred solution of (S)-(-)-4-(3-cyanophenyl)-2- trifluoromethyl-5-oxo-1,4,5,6,7,8-hexahydroquinoline-3-carboxylic acid (50.5g, 139.4 mmol) in N-methylpyrrolidin-2-one (325mL) was heated rapidly (heating mantle) over 20 minutes to 180°C (internal thermometer), then maintained at that temperature for an additonal 20 minutes. The cooled reaction mixture was poured into water (1200mL) and extracted twice with ethyl acetate. The combined extracts were washed twice with water, dried (HgSO₄), filtered and the solvent removed to yield an off-white solid. Chromatography (eluent: methylene chloride/ethyl acetate 85:15) and recrystallisation from acetonitrile provided the title compound (32.85g, 74%) as a white solid which was identical to the product of Example 1.

### Example 3

The intermediate (±)-isobornyl 4,4,4-trifluoroacetoacetate (of Example la) was prepared on a larger scale as follows:

A stirred mixture of ethyl 4,4,4-trifluoroacetoacetate (2.75Kg, 14.91 moles) and (±)-isoborneol (1.53Kg, 9.93 moles) was stirred at 105°C (internal thermometer) for 20 hours under a distillation head which allowed ethanol to distill off. The temperature was then increased gradually over 10 hours to 155°C to distill the residual ethanol; a total of 650 mL (114% of theory) was collected. The remaining mixture was then fractionated at reduced pressure to yield (±)-isobornyl 4,4,4-trifluoroacetoacetate as a colorless oil (2.21Kg 76%) which was identical to the product of Example 1a.

### Example 4

The intermediate (±)-isobornyl 4-(3-cyanophenyl)-2- trifluoromethyl-2-hydroxy-5-oxo-1,2,3,4,5,6,7,8-octahydroquinoline- 3-carboxylate (of Example 1b) was prepared on a larger scale as follows: A stirred mixture of (±)-isobornyl 4,4,4- trifluoroacetoacetate (830.6g 2.86 moles), 1,3-cyclohexanedione (320.7g 2.86 moles), 3-cyanobenzaldehyde (375.0 g 2.86 moles), and ammonium acetate (551.0g 7.15 moles) in ethanol (15.0 L) was refluxed for 8 hours. The cooled mixture was filtered to remove 9-(3-cyanophenyl)-3,4,6,7,9,10 -hexahydro-1,8-(2H,5H)-acridinedione. The filtrate was concentrated to half volume in vacuo. A first crop of desired product was collected by filtration and washed with ethyl ether (657.0g, 44.5%). The filtrates and washes were concentrated in vacuo, and the residue triturated with ethyl ether and filtered to provide a second crop of product (225.0g, 15.2%). This combined material was used without further purification. A sample was purified by chromatography (eluent: ethyl acetate/hexane 7:3) to provide (±)-isobornyl 4-(3-cyanophenyl)-2-trifluoromethyl-2-hydroxy-5-oxo- 1,2,3,4,5,6,7,8-octahydroquinoline-3-carboxylate as a white solid which was identical to that prepared in Example 1b.

### Example 5

### Preparation of 4-(3-Cyanophenyl)-2-trifluoromethyl-5-oxo-1,4,5,6,7,8- hexahydroquinoline-3-carboxylic acid (of Example 1c) on a larger scale was carried out as follows:

A stirred mixture of (±)-isobornyl 4-(3-cyanophenyl)- 2-trifluoromethyl-2-hydroxy-5-oxo-1,2,3,4,5,6,7,8-octahydroquinoline- 3-carboxylate (800.0g 1.55 moles), p-toluenesulfonic acid (148.0g, 0.78 moles) and glacial acetic acid (7.5L) was heated (heating mantle) over 1 hour 15 minutes to 100°C. The mixture was maintained at a temperature of 102-104°C for 6 hours, at which point TLC (silica gel-ethyl acetate/hexane 6:4) indicted that the reaction was complete. To minimise decarboxylation, only enough heat was applied to the reaction mixture so as to maintain the temperature at a maximum of 104°C. After removal of solvent, the residue was partitioned between ethyl acetate and water. The ethyl acetate was washed with water, separated and extracted three times with saturated aqueous sodium bicarbonate. The stirred combined sodium bicarbonate extracts were cooled in an ice bath and concentrated hydrochloric acid was added dropwise until the solution was strongly acidic. The mixture was extracted with ethyl ether and the combined extracts were dried (MgSO₄), filtered, and concentrated in vacuo to give a yellow foam. Trituration with methylene chloride/hexane yielded the carboxylic acid (289.0g, 51.5%) as a light yellow solid. The material was identical by NMR and tlc (silica gel - 10% methanol in chloroform containing a few drops of acetic acid) to the material described and characterized in sub-part i. of Example 1.

The ethyl acetate layer was dried (MgSO₄), filtered and evaporated in vacuo, to obtain impure (±) 4-(3-cyanophenyl) -2-trifluoromethyl-4,6,7,8-tetrahydro-5(1H)-quinolone (490g) as a waxy light yellow solid. This material is hereafter referred to as the Racemate.

### Example 6

The intermediate (S)-(-)-4-(3-cyanophenyl)-2-trifluoromethyl-5- oxo-1,4,5,6,7,8-hexahydroquinoline-3-carboxylic acid (of Example le) was prepared on a larger scale as follows:
To a cooled (ice bath) stirred slurry of S-(-)-a-methylbenzylamine (S)-(-)-4-(3-cyanophenyl)-2-trifluoromethyl-5-oxo-1,4,5,6,7,8-hexahydroquinoline-3-carboxylic acid salt (68.0g, 140.6 mmol) in water (700 mL) was added concentrated hydrochloric acid dropwise until the mixture was strongly acidic. The mixture was extracted four times with ethyl ether, and the combined extracts dried (MgSO₄,), filtered and the solvent removed to yield a yellow foam (50.6g, 99%). Trituration with methylene chloride/hexane provided a pure sample of the carboxylic acid as a pale yellow solid which was identical to the product of Example le.

### Example 7

The title compound (S)-(-)-4-(3-Cyanophenyl)-2-trifluoromethyl-4,6,7,8-tetrahydro-5(1H)-quinolone was isolated from purified Racemate by preparative chiral chromatography.

Crude Racemate (2.95Kg - material pooled from 6 equal sized batch runs isolated as described in Example 5 was purified by chromatography (eluent: methylene chloride/ethyl acetate 85:15) to provide purified Racemate (580.8g) which was separated as described below. Recovery of this material during this initial purification was not optimised.

Preparative chiral chromatography of the purified Racemate (580.8g) was carried out on a CHIRALPACK^{R}AD™ (trademark) column (Amylose support, 10X50 cm. eluent: hexane/ethanol 85:15). The sample stock solution was prepared by initially dissolving the compound in ethanol, then diluting the solution with hexane until a final solvent composition equivalent to that of the eluent was achieved. The final sample concentration of the stock solution produced was 6g/liter. Each chromatographic run was conducted by injecting 500 mL of the stock solution and eluting with a flow rate of 200 ml/minute. The (S)-(-)-enantiomer (title compound) was the first peak to elute from the column, the (+)-enantiomer eluting second. Evaporation of the eluate in vacuo recovered the (S)-(-)-enantiomer (281.0g, 99.7% ee, 98.6% chemical purity) as an off-white solid. Recrystallisation from acetonitrile (1000 mL) provided 261.7g of the title compound as a white crystalline solid, identical in every respect to the material prepared as described Example 1.

### Example 8. The following illustrate representative pharmaceutical dosage forms containing the compound, (hereafter referred to as "compound X"), for therapeutic or prophylactic use in humans:

(a) Tablet

| | mg/tablet |
|---|---|
| Compound X | 50.0 |
| Mannitol, USP | 223.75 |
| Croscarmellose sodium | 6.0 |
| Maize starch | 15.0 |
| Hydroxypropylmethylcellulose (HPMC), USP | 2.25 |
| Magnesium stearate | 3.0 |

(b) Capsule

| | |
|---|---|
| Compound X | 10.0 |
| Mannitol, USP | 488.5 |
| Croscarmellose sodium | 15.0 |
| Magnesium stearate | 1.5 |

The above formulations may be obtained by conventional procedures well known in the pharmaceutical art. The tablets may be enteric coated by conventional means, for example to provide a coating of cellulose acetate phthalate.

### Formulae

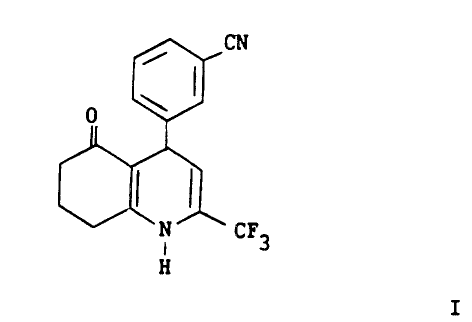

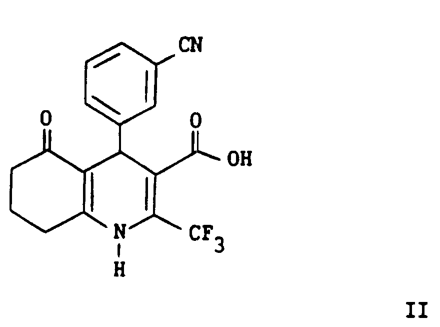

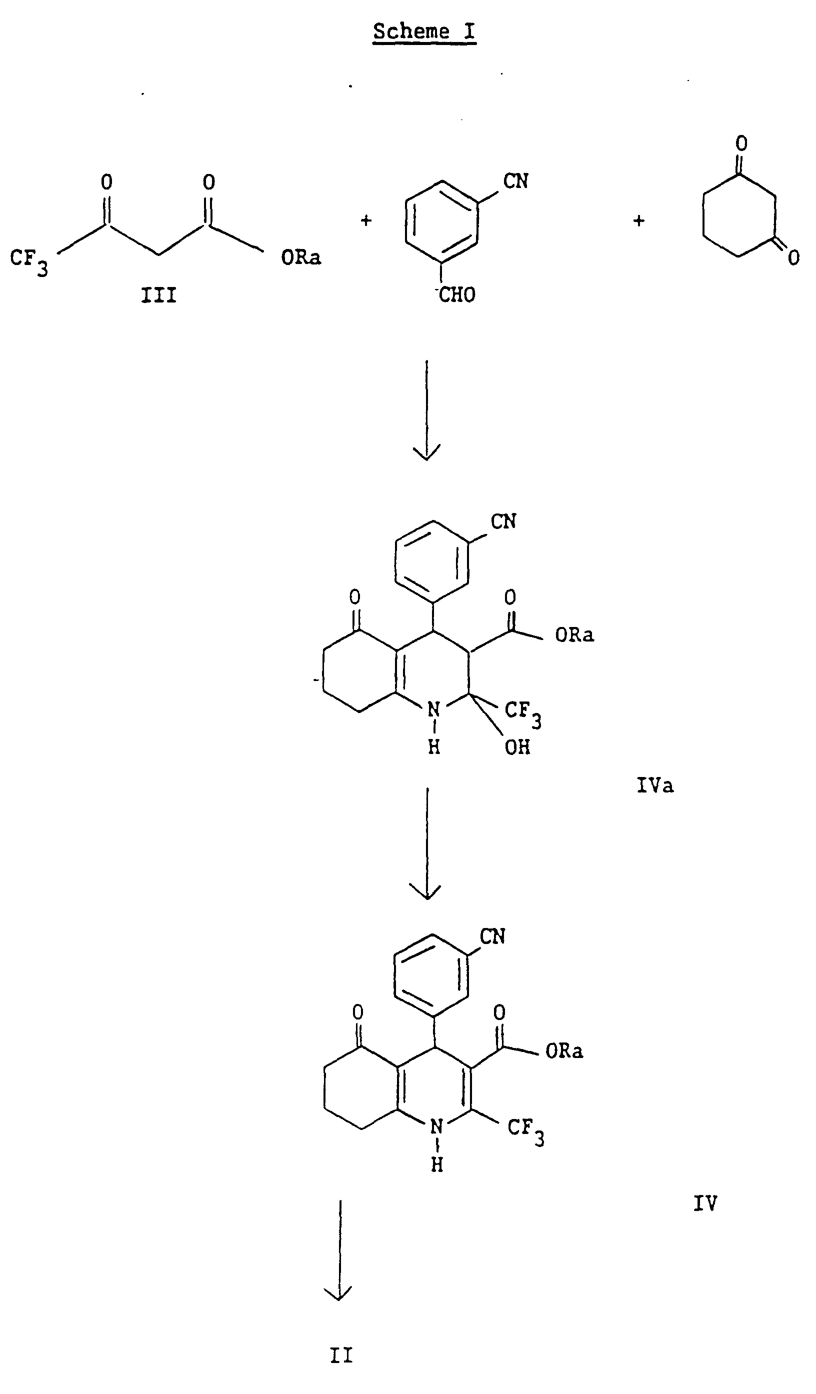

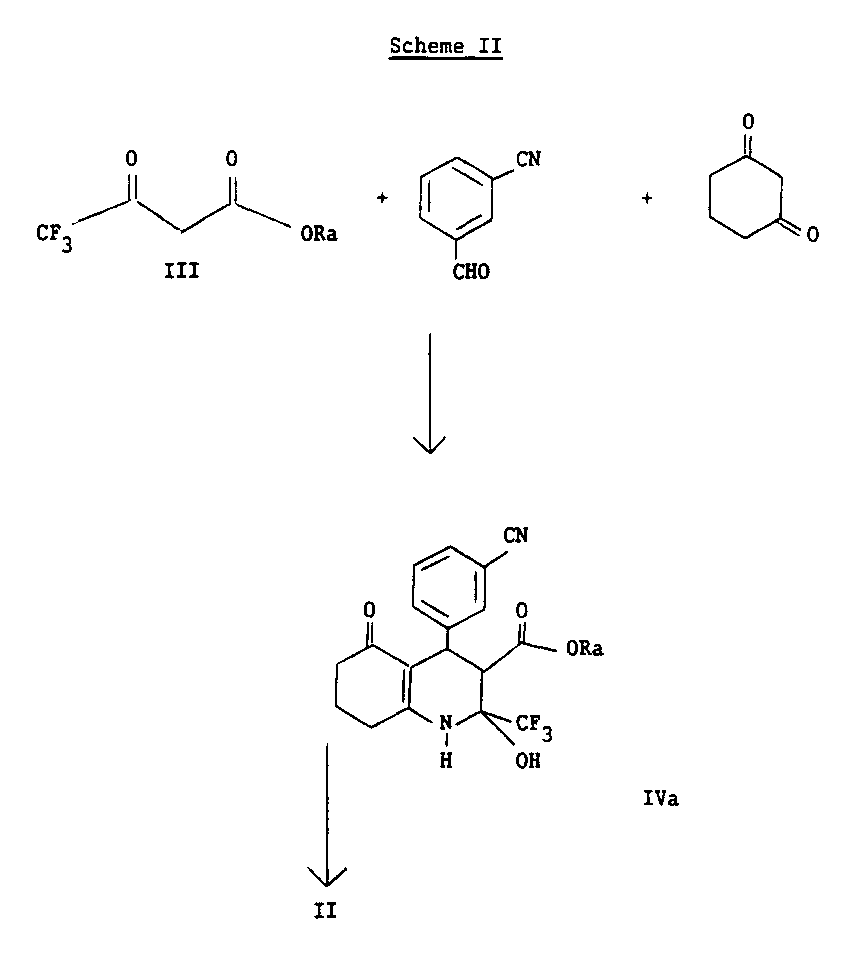

## Claims

1. (S)-(-)-2-trifluoromethyl-4-(3-cyanophenyl)-4,6,7,8-tetrahydro-5(1H)-quinolone and pharmaceutically acceptable salts thereof.

2. Pharmaceutical composition comprising the compound as defined in claim 1 or a pharmaceutically acceptable salt thereof, and a pharmaceutically acceptable diluent or carrier.

3. Process for the preparation of (S)-(-)-2-trifluoromethyl-4-(3-cyanophenyl)-4,6,7,8-tetrahydro-5(1H)quinolone comprising decarboxylation of (S)-(-)-2-trifluoromethyl-4-(3-cyanophenyl)-5-oxo-1,4,5,6,7,8-hexahydroquinoline-3-carbo xylic acid.

4. Process according to claim 3 wherein the decarboxylation is carried out at a temperature in the range of from 190°C to 220°C.

5. Process according to claim 3 wherein the decarboxylation is carried out in an inert solvent of appropriate boiling point.

6. Process according to claim 5 wherein the inert solvent is N-methylpyrrolidin-2-one.

7. Process according to claim 3 wherein the decarboxylation is carried out in the presence of an acid catalyst.

8. Process according to claim 7 wherein the decarboxylation is carried out at a temperature in the range of from 90°C to 120°C.

9. Process according to claim 7 wherein the acid catalyst is concentrated sulphuric acid or p-toluenesulphonic acid.

10. Process according to claim 7 wherein acid catalyzed decarboxylation is carried out in the presence of solvent selected from alcohols, dimethylsulfoxide, aromatic hydrocarbons, ethers, and N-methylpyrrolidin-2-one.

11. Process for the preparation of (S)-(-)-2-trifluoromethyl-4-(3-cyanophenyl)-4,6,7,8-tetrahydro-5(1H)quinolone which comprises the steps of:
(i) reacting an acetoacetic ester of formula III or a hemiacetal thereof: 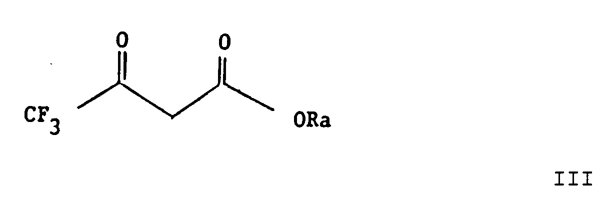 in which ORa is an alcohol residue, which forms an ester that can be cleaved under mild basic conditions or which forms an ester that can be cleaved under acidic conditions, with ammonium acetate, 3-cyanobenzaldehyde and 1,3-cyclohexanedione to give a compound of formula IVa: 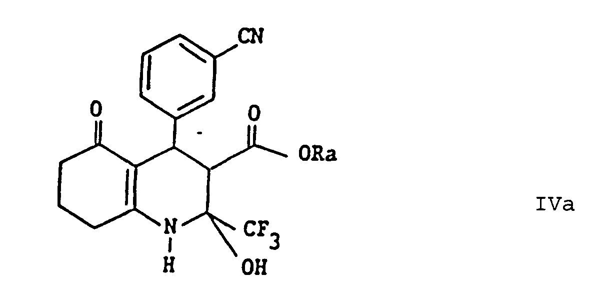 and
(ii) alternately:
dehydrating the compound of formula IVa to a compound of formula IV: 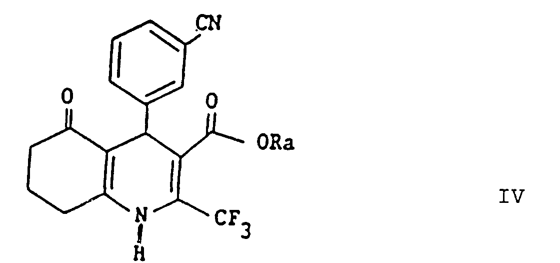
and saponifying the compound of formula IV to give an acid of formula II, 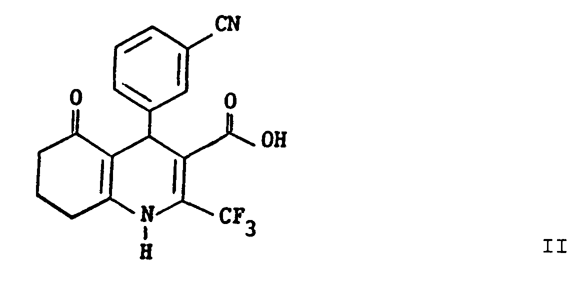
or, subjecting the compound of formula IVa to acid catalysed dehydration and hydrolysis to give an acid of formula II;
(iii) resolving the compound of formula II by recrystallisation as the S-(-)-α-methylbenzylamine salt from solvent; and
(iv) decarboxylating the resolved compound of formula II.

12. Process for preparation according to claim 11 wherein ORa is an alcohol residue which forms an ester that can be cleaved under mild basic conditions.

13. Process according to claim 12 wherein Ra is 2-cyanoethyl.

14. Process for preparation according to claim 11 wherein ORa forms an ester that can be cleaved under acidic conditions.

15. Process according to claim 14 wherein Ra is iso-bornyl.

16. Use of a compound as claimed in claim 1 in the manufacture of a medicament for the treatment of urinary incontinence.

17. Use of a compound as claimed in claim 1 in the manufacture of a medicament for the treatment of asthma.

## Patentansprüche

1. (S)-(-)-2-Trifluormethyl-4-(3-cyanophenyl)-4,6,7,8-tetrahydro-5(1H)-chinolon und pharmazeutisch unbedenkliche Salze davon.

2. Pharmazeutische Zusammensetzung, enthaltend die in Anspruch 1 definierte Verbindung oder ein pharmazeutisch unbedenkliches Salz davon, und ein pharmazeutisch unbedenkliches Verdünnungsmittel oder einen pharmazeutisch unbedenklichen Träger.

3. Verfahren zur Herstellung von (S)-(-)-2-Trifluormethyl-4-(3-cyanophenyl)-4,6,7,8-tetrahydro-5(1H)-chinolon, bei dem man (S)-(-)-2-Trifluormethyl-4-(3-cyanophenyl)-5-oxo-1,4,5,6,7,8-hexahydrochinolin-3-carbonsäure decarboxyliert.

4. Verfahren nach Anspruch 3, bei dem man die Decarboxylierung bei einer Temperatur im Bereich von 190°C bis 220°C durchführt.

5. Verfahren nach Anspruch 3, bei dem man die Decarboxylierung in einem inerten Lösungsmittel mit geeignetem Siedepunkt durchführt.

6. Verfahren nach Anspruch 5, bei dem man als inertes Lösungsmittel N-Methylpyrrolidin-2-on einsetzt.

7. Verfahren nach Anspruch 3, bei dem man die Decarboxylierung in Gegenwart eines sauren Katalysators durchführt.

8. Verfahren nach Anspruch 7, bei dem man die Decarboxylierung bei einer Temperatur im Bereich von 90°C bis 120°C durchführt.

9. Verfahren nach Anspruch 7, bei dem man als sauren Katalysator konzentrierte Schwefelsäure oder p-Toluolsulfonsäure einsetzt.

10. Verfahren nach Anspruch 7, bei dem man die säurekatalysierte Decarboxylierung in Gegenwart von Lösungsmittel, das unter Alkoholen, Dimethylsulfoxid, aromatischen Kohlenwasserstoffen, Ethern und N-Methylpyrrolidin-2-on ausgewählt wird, durchführt.

11. Verfahren zur Herstellung von (S)-(-)-2-Trifluormethyl-4-(3-cyanophenyl)-4,6,7,8-tetrahydro-5(1H)-chinolon, bei dem man:
(i) einen Acetessigsäureester der Formel III oder ein Hemiacetal davon: 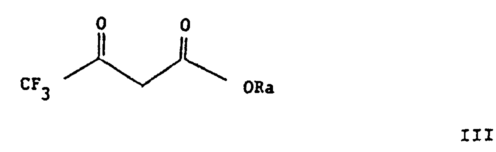 worin ORa für einen Alkoholrest steht, der einen unter milden basischen Bedingungen spaltbaren Ester oder einen unter sauren Bedingungen spaltbaren Ester bildet, mit Ammoniumacetat, 3-Cyanobenzaldehyd und 1,3-Cyclohexandion zu einer Verbindung der Formel IVa: 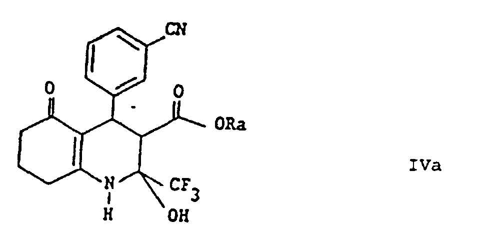 umsetzt und
(ii) wahlweise:
die Verbindung der Formel IVa zu einer Verbindung der Formel IV: 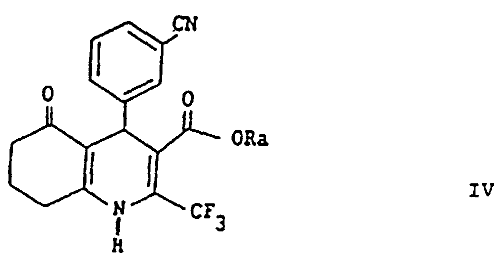
dehydratisiert und die Verbindung der Formel IV zu einer Säure der Formel II 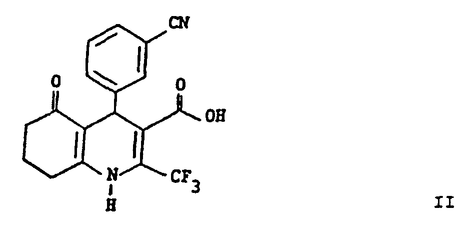
verseift oder die Verbindung der Formel IVa durch säurekatalysierte Dehydratisierung und Hydrolyse in eine Säure der Formel II überführt;
(iii) die Verbindung der Formel II durch Umkristallisation als (S)-(-)-α-Methylbenzylamin-Salz aus Lösungsmittel einer Racematspaltung unterwirft und
(iv) die racematgespaltene Verbindung der Formel II decarboxyliert.

12. Herstellungsverfahren nach Anspruch 11, bei dem ORa für einen Alkoholrest steht, der einen unter milden basischen Bedingungen spaltbaren Ester bildet.

13. Verfahren nach Anspruch 12, bei dem Ra für 2-Cyanoethyl steht.

14. Herstellungsverfahren nach Anspruch 11, bei dem ORa einen unter sauren Bedingungen spaltbaren Ester bildet.

15. Verfahren nach Anspruch 14, bei dem Ra für Isobornyl steht.

16. Verwendung einer Verbindung nach Anspruch 1 bei der Herstellung eines Arzneimittels zur Behandlung von Harninkontinenz.

17. Verwendung einer Verbindung nach Anspruch 1 bei der Herstellung eines Arzneimittels zur Behandlung von Asthma.

## Revendications

1. (S)-(-)-2-trifluorométhyl-4-(3-cyanophényl)-4,6,7,8-tétrahydro-5(1H)-quinolone et sels pharmaceutiquement acceptables de celle-ci.

2. Composition pharmaceutique comprenant le composé tel que défini à la revendication 1 ou un sel pharmaceutiquement acceptable de celui-ci, et un diluant ou support pharmaceutiquement acceptable.

3. Procédé de préparation de la (S)-(-)-2-trifluorométhyl-4-(3-cyanophényl)-4,6,7,8-tétrahydro-5(1H)-quinolone, comprenant la décarboxylation de l'acide (S)-(-)-2-trifluorométhyl-4-(3-cyanophényl)-5-oxo-1,4,5,6,7,8-hexahydroquinoéline-3-carboxylique,

4. Procédé selon la revendication 3, dans lequel la décarboxylation est réalisée à une température dans la gamme de 190°C à 220°C.

5. Procédé selon la revendication 3, dans lequel la décarboxylation est réalisée dans un solvant inerte ayant un point d'ébullition approprié.

6. Procédé selon la revendication 5, dans lequel le solvant inerte est la N-méthylpyrrolidin-2-one.

7. Procédé selon la revendication 3, dans lequel la décarboxylation est réalisée en présence d'un catalyseur acide.

8. Procédé selon la revendication 7, dans lequel la décarboxylation est réalisée à une température dans la gamme de 90°C à 120°C.

9. Procédé selon la revendication 7, dans lequel le catalyseur acide est de l'acide sulfurique concentré ou de l'acide p-toluènesulfonique.

10. Procédé selon la revendication 7, dans lequel la décarboxylation à catalyse acide est réalisée en présence d'un solvant choisi parmi des alcools, le diméthylsulfoxy, des hydrocarbures aromatiques, des éthers et la N-méthylpyrrolidin-2-one.

11. Procédé de préparation de la (S)-(-)-2-trifluorométhyl-4-(3-cyanophényl)-4,6,7,8-tétrahydro-5(1H)-quinolone, comprenant les étapes consistant à :
(i) faire réagir un ester acétoacétique de formule III ou un hémiacétal de celui-ci : 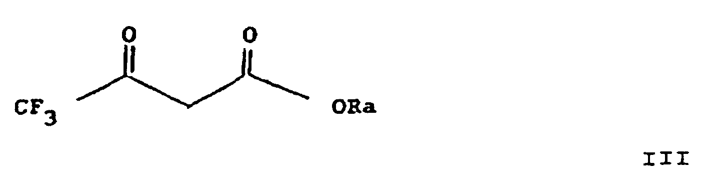 dans laquelle ORa est un résidu alcool, qui forme un ester pouvant être clivé dans des conditions basiques douces ou qui forme un ester pouvant être clivé dans des conditions acides, avec l'acétate d'ammonium, le 3-cyanobenzaldéhyde et la 1,3-cyclohexanedione pour donner lieu à un composé de formule IVa : 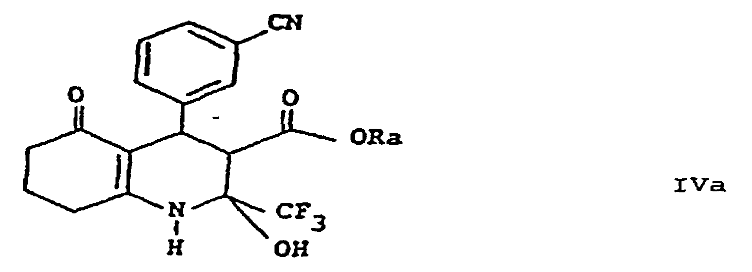 et
(ii) en variante :
déshydrater le composé de formule IVa pour donner un composé de formule IV : 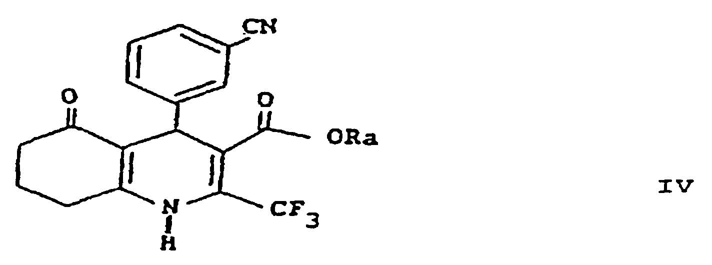
et saponifier le composé de formule IV pour donner un composé de formule II, 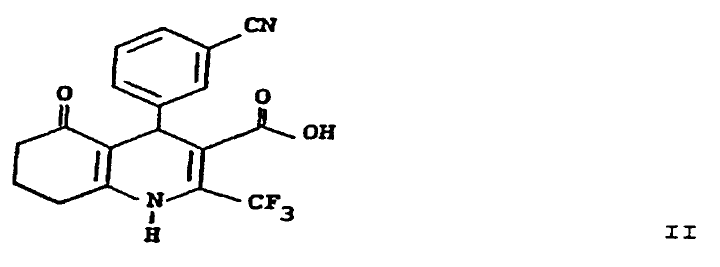
ou soumettre le composé de formule IVa à une déshydratation à catalyse acide et une hydrolyse pour donner un acide de formule II ;
(iii) dédoubler le composé de formule II par recristallisation sous forme du sel de S-(-)-α-méthylbenzylamine dans du solvant ; et
(iv) décarboxyler le composé dédoublé de formule II.

12. Procédé de préparation selon la revendication 11, dans lequel ORa est un résidu alcool qui forme un ester pouvant être clivé dans des conditions basiques douces.

13. Procédé selon la revendication 12, dans lequel Ra est un 2-cyanoéthyle.

14. Procédé de préparation selon la revendication 11, dans lequel ORa forme un ester pouvant être clivé dans des conditions acides.

15. Procédé selon la revendication 14, dans lequel Ra est un isobornyle.

16. Utilisation d'un composé selon la revendication 1 pour l'élaboration d'un médicament destiné au traitement de l'incontinence urinaire.

17. Utilisation d'un composé selon la revendication 1 pour l'élaboration d'un médicament destiné au traitement de l'asthme.
